**Europäisches Patentamt**

**European Patent Office**

**Office européen des brevets**

(19)

(11) Publication number: **0 145 430 B1**

# EUROPEAN PATENT SPECIFICATION

(12)

(45) Date of publication of patent specification: **29.05.91**  (51) Int. Cl.⁵: **A61F 11/04, H04B 5/00**

(21) Application number: **84308374.2**

(22) Date of filing: **03.12.84**

(54) **Signal transmission system.**

(30) Priority: **09.12.83 US 559923**

(43) Date of publication of application:
**19.06.85 Bulletin 85/25**

(45) Publication of the grant of the patent:
**29.05.91 Bulletin 91/22**

(84) Designated Contracting States:
**AT CH DE FR GB LI**

(56) References cited:
**US-A- 4 223 679**

**IEEE TRANSACTIONS ON BIO-MEDICAL EN-
GINEERING, vol. BME-16, no. 3, July 1969,
pages 177-183; IEEE, New York, US
R.KADEFORS et al.: "Energizing implantable
transmitters by means of coupled induc-
tance coils."**

(73) Proprietor: **Cochlear Corporation
61 Inverness Drive East
Englewood Colorado 80112(US)**

(72) Inventor: **Sontag, Hugh D. c/o Minnesota Min-
ing and
Manufact. Company 2501 Hudson Road P.O.
Box 33427
St. Paul Minnesota 55133-3427(US)**

(74) Representative: **Baillie, Iain Cameron et al
c/o Ladas & Parry Isartorplatz 5
W-8000 München 2(DE)**

## Description

Technical Field

The present invention relates generally to signal transmission systems and more particularly to signal transmission systems which transmit a signal across a boundary with close proximity between transmitting and receiving antennas.

Background Art

The environment where the signal transmission system of the present invention operates is in a system where the transmission is over a short distance across a barrier. An example of this environment is the use of a signal transmission system with a hearing prosthetic device in which a receiver is implanted within the body to drive an electrode to electrically stimulate the auditory nerve. A transmitting antenna is positioned in close proximity to the receiving antenna but across the cutaneous barrier. Signals representing sound are then transmitted across the cutaneous boundary to the receiver for ultimate delivery to the electrode. The signal transmission system then avoids the use of a percutaneous plug and its attendant potential for infection.

Typically, the transmitter and receiver of the signal transmission system use resonant LC antennas to transmit and receive a small amount of electrical energy over a short distance, e.g. centimeters over the skin boundary.

Signal transmission systems are in use in which the transmitter utilizes a separate oscillator and antenna. In this situation, the resonant frequencies of each are separate and adjustable. The frequency of the oscillator is adjusted to the unloaded resonant frequency of the antenna. The spacing between the transmitter and receiver is then carefully adjusted to be the distance at which "critical coupling" occurs. The "critical coupling" phenomenon is well known in the art. At the point of "critical coupling" a minimum rate of change of the output level of the receiver with respect to variations in the antenna to antenna spacing is observed. Near "critical coupling" and over a small spacing variation, the output of the receiver is relatively invariant.

However, if larger variations in spacing occur between the transmitting antenna and the receiving antenna, the signal transmitted to the receiver will be diminished.

Similarly, if mismatches are evident between the frequency of oscillation and the resonant frequency of its antenna, the signal transmitted to the receiver will be diminished.

There is disclosed in IEEE Transactions on Bio-Medical Engineering, Vol. BME-16, No. 3, July 1969, pages 177-183 an Article by R. Kadefors et al which is directed toward energising an implantable transmitter by coupling energy to the transmitter through an inductive link. Some of the implanted transmitters may be sensitive to the excitation voltage applied but the article notes that severe difficulties can be expected to arise if voltage regulation is not compensated for variation in coil separation but no teaching is made as to overcome this problem. Also the Article discloses that the optimum frequency of transmission changes as the separation between coils varies. Also the Article notes that variations in transmission efficiency caused, for example, by diminishing the coil separation, may result in sudden changes in the maximum value of the voltage produced. US-A-4,223,679 relates to a telemetry system for use in a living tissue stimulator system in which an externally located oscillator is controlled by impedance changes in an impedance reflecting circuit located in an implantable tissue stimulator. The disclosure of this reference is therefore to change the impedance of a coil located in a body to cause frequency or amplitude modulation of an oscillator having a coil located outside the body. The variations in the oscillator located outside the body are then de-modulated and a signal is recovered. By such expedient, causing variations in impedance of the coil located within the body, coupled to the transmitter coil outside the body, results in the ability to telemeter information out of the body. In distinction to these references, the present invention seeks to provide a signal transmission system that is relatively independent of the distance between the transmitting and receiving antennas.

Disclosure of Invention

According to this invention there is provided a signal transmission system for use in transmitting a signal across a barrier, having a radio frequency receiver for use on one side of said barrier; a receiving resonant antenna for use on said one side of said barrier being operatively coupled to said radio frequency receiver, said receiving resonant antenna having a resonant frequency; a transmitting resonant antenna for use on the opposite side of said barrier nearby said receiving resonant antenna, said transmitting resonant antenna having a resonant frequency; said resonant frequency of said receiving resonant antenna being relatively close to said resonant frequency of said transmitting resonant antenna; and having a radio frequency transmitter for use on said opposite side of said barrier being operatively coupled to said transmitting resonant antenna, said radio frequency transmitter having an oscillator having a frequency of oscillation driving a resonant load where said resonant load includes said transmitting resonant antenna inductively coupled with said receiving resonant antenna; characterised by means for tracking the frequency of oscillation of said oscillator tracking to the resonant frequency of said resonant load and maintaining a virtually identical amplitude of the signal provided to the receiver by said transmission system; whereby signal transmission by said signal transmission system is relatively independent of the distance between said transmitting resonant antenna and of said receiving resonant antenna, of radial misalignment between said transmitting resonant antenna and said receiving resonant antenna, and resonant frequency mismatch of said transmitting resonant antenna and said receiving resonant antenna.

In a preferred embodiment, the signal transmission system is adapted for use in a transcutaneous environment wherein the receiving resonant antenna and radio frequency receiver are positioned for subcutaneous use and the transmitting resonant antenna and the radio frequency transmitter are positioned for supercutaneous use.

A signal transmission system constructed in this manner provides a transmitter which is self-resonant, i.e. which oscillates at the resonant frequency of the resonant load which includes the tranmsitting and receiving antennas. This results in the signal level at the receiver being virtually constant over a much wider range of distance variations between the transmitting antenna and the receiving antenna, both axially and laterally. The reason this stability in signal level from the receiver is achieved is due to the effect of the receiving antenna on the resonant frequency of the transmitting antenna coupled with the dependence of the frequency of oscillation of the transmitter on the resonance of the resonant load. This effect causes the operating frequency of the signal transmission system to change such that the output voltage from the receiver stays virtually constant.

## Brief Description of Drawings

The foregoing advantages, construction and operation of the signal transmission system of the present invention will become more readily apparent from the following description and accompanying drawings in which:

Figure 1 is a block diagram of a signal transmission system of the present invention;

Figure 2 is a schematic diagram of the transmitter and transmitting antenna;

Figure 3 is a schematic diagram of the receiver and the receiving antenna;

Figure 4 is a graph illustrating prior art variations in receiving level versus antenna spacing; and

Figure 5 is a graph illustrating the level of amplitude of received signal as a function of frequency.

## Detailed Description

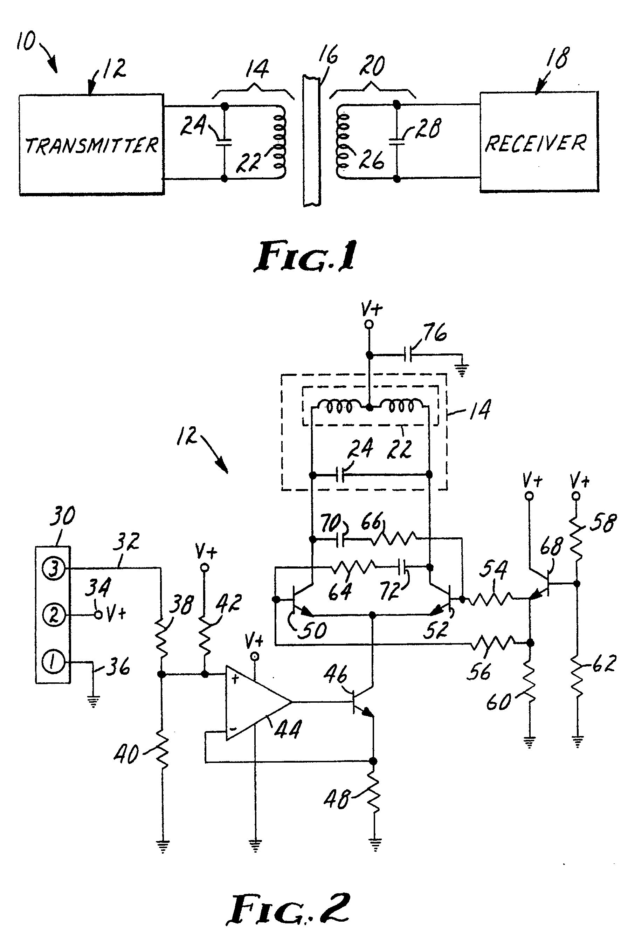

Figure 1 illustrates a block diagram of the signal transmission system 10 of the present invention. A transmitter 12 is operatively coupled to a transmitting antenna 14 positioned on one side of a boundary or barrier 16. On the opposite side of the barrier or boundary 16 a receiver 18 is operatively coupled to a receiving antenna 20. In a preferred embodiment, the transmitting antenna consists of a resonant LC circuit involving inductor 22 and capacitor 24. Similarly, in a preferred embodiment, receiving antenna 20 also comprises an LC circuit consisting of inductor 26 and capacitor 28. While both the transmitting antenna 14 and the receiving antenna 20 are shown as tank circuits having a parallel connection of an inductor (22, 26) and a capacitor (24, 28, respectively) various other combinations of capacitive and inductive elements can be formed to comprise a resonant circuit such as, and including, the series combination of an inductor and a capacitor.

The receiving resonant antenna 20 has a resonant frequency which is dependent upon the tank circuit comprising inductor 26 and capacitor 28. Similarily, transmitting antenna 14, in the absence of other factors, has a resonant frequency which is dependent upon the tank circuit comprising inductor 22 and capacitor 24. Inductor 22 of the transmitting resonant antenna 14 and inductor 26 of the receiving resonant antenna 20

3

are positioned for relatively close lateral alignment along barrier 16 and are also positioned within fairly close proximity across barrier 16, i.e. axial alignment. In a preferred embodiment, the axial spacing between the transmitting antenna 14 and the receiving antenna 20 is chosen such that axial spacing is less than the critical coupling distance. The resonant frequency of the transmitting antenna 14 is chosen so that it is relatively close to the resonant frequency of the receiving antenna 20. Relatively close, for purposes of this discussion, generally means in the range of being within ten percent. Certainly variations in the resonant frequencies of transmitting antenna 14 and receiving antenna 20 can be twenty percent and even greater. However, it is generally preferred that the resonant frequencies of the transmitting antenna 14 and the receiving antenna 20 be within ten percent of each other. Transmitter 12 has an oscillator which drives a resonant load which includes transmitting antenna 14 and its inductive coupling with receiving antenna 20. Further, the frequency of oscillation of the oscillator of transmitter 12 is at least partially determined by the frequency of the resonant load, i.e. the combination of the resonant frequency of transmitting antenna 14 along with the effects of the close proximity of receiving antenna 20. In a preferred embodiment, the frequency of oscillation of the oscillator of transmitter 12 is primarily determined by the resonance of the resonant load and in a still preferred embodiment, is identical to the resonant frequency of the resonant load.

Figure 2 illustrates a schematic diagram of the transmitter 12 in conjunction with the transmitting antenna 14. Transmitting antenna 14 consists of a tank circuit comprising inductor 22 and capacitor 24 as illustrated in Figure 1. The signal to the transmitter 12 is provided through junction block 30 which provides a signal 32, a voltage source 34 and the ground return for the voltage source 36. The signal 32 drives resistor 38, resistor 40, resistor 42, operational amplifier 44 to feed transistor 46 and resistor 48 forms a constant current source, controlled by signal 32. Transistor 46 of the constant current source feeds transistors 50 and 52 which together with associated resistors 54, 56, 58, 60, 62, 64 and 66, transistor 68 and capacitor 70 and 72 form the oscillator circuit of the transmitter 12. This oscillator drives antenna 14 (in the form of inductor 22 along with capacitor 24) to form, in the absence of other circumstances, the resonant load of the oscillator of the transmitter 12. Capacitor 76 is a decoupling capacitor. A distinguishing feature of transmitter 12 is that it contains one oscillator whose frequency of oscillation is dependent upon the resonant load which that oscillator drives.

Figure 3 represents a schematic diagram of receiver 18 and receiving antenna 20. Again, as in Figure 1, receiving antenna 20 consists of inductor 26 parallel coupled to capacitor 28. Of course, various other tank circuits are envisioned for this receiving antenna 20. Receiving antenna 20 drives diode 82, capacitor 84 with resistor 86, which together with DC blocking capacitor 88 demodulate the signal for transmission to an electrode or other load for the transmitted signal. In a preferred embodiment, the load is an electrode which is implanted in the ear as an auditory hearing prosthesis to stimulate body tissue and, in particular, the auditory nerve.

Table 1 illustrates examples of values for the components utilized in the transmitter described in Figure 2 and the receiver described in Figure 3.

## TABLE I

| Reference Numeral | Component | Value | Vendor |
|---|---|---|---|
| 22 | Inductor | 400 nanohenries | |
| 24 | Capacitor | 310 picofarads | |
| 26 | Inductor | 1.76 microhenries | |
| 28 | Capacitor | 100 picofarads | |
| 38 | Resistor | 16 kilohms | |
| 40 | Resistor | 4 kilohms | |
| 42 | Resistor | 1.6 megohms | |
| 44 | Operational Amplifier | CA 3420 | RCA |
| 46 | Transistor | MPS-C6595 | Motorola |
| 48 | Resistor | 10 ohms | |
| 50 | Transistor | MPS-C6595 | Motorola |
| 52 | Transistor | MPS-C6595 | |
| 54 | Resistor | 2.2 kilohms | |
| 56 | Resistor | 2.2 kilohms | |
| 58 | Resistor | 33 kilohms | |
| 60 | Resistor | 10 kilohms | |
| 62 | Resistor | 24 kilohms | |
| 64 | Resistor | 2.2 kilohms | |
| 66 | Resistor | 2.2 kilohms | |
| 68 | Transistor | MPS-C6595 | Motorola |
| 70 | Capacitor | 0.01 microfarads | |
| 72 | Capacitor | 0.01 microfarads | |
| 76 | Capacitor | 0.1 microfarads | |
| 82 | Diode | 1N5711 | |
| 84 | Capacitor | 1,000 picofarads | |
| 86 | Resistor | 3.3 kilohms | |
| 88 | Capacitor | 0.1 microfarads | |

Figure 4 is a graph in which the spacing between the antenna coils of the transmitting antenna 14 and the receiving antenna 20 is plotted against the amplitude of the voltage of the received signal at the receiver 18. As can be seen in the graph, there is a spacing 94 between the coils of the transmitting antenna 14 and the receiving antenna 20 at which the amplitude of the voltage of the received signal is at a maximum. Also note that the slope of the curve is at a minimum at that particular spacing. It is at this spacing between the coils of the transmitting antenna 14 and the receiving antenna 20 that variations in the antenna spacing provide a minimal effect on the voltage amplitude of the received signal. Thus, the point 94 illustrated in Figure 4 is the point of "critical coupling" between the transmitting antenna 14 and the receiving antenna 20.

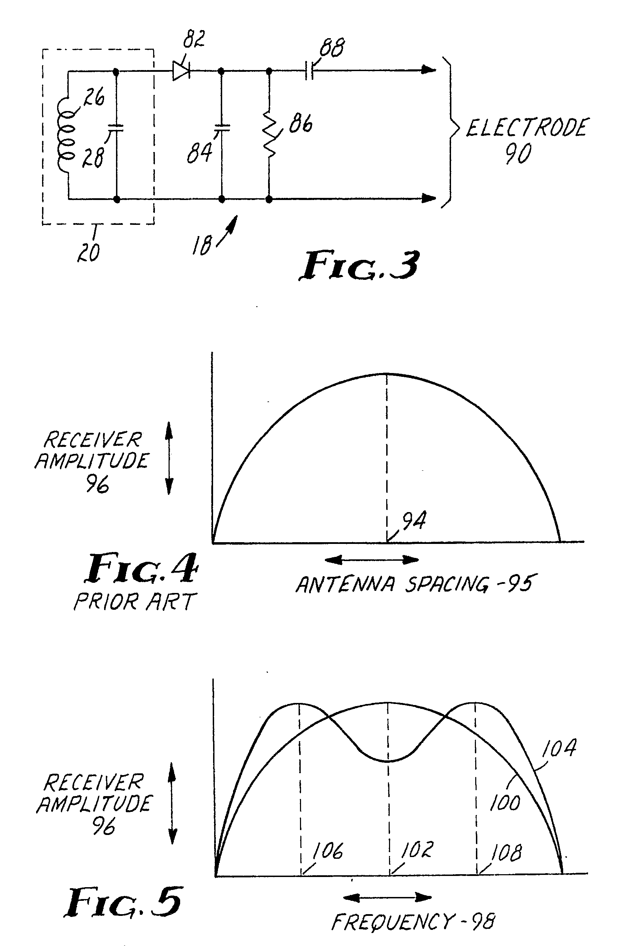

Figure 5 illustrates a graph in which the amplitude of the received signal 96 is plotted against the frequency 98 of operation of the transmitter oscillator. When the coils of the transmitting antenna 14 and the receiving antenna 20 are critically aligned and spaced and when the resonant frequencies of the transmitting antenna 14 and the receiving antenna 20 are identical, line 100 on the graph illustrates the effect of variations in the frequency of operation of the signal transmission system upon the amplitude of the received signal 96. From the graph it can be seen that there is a preferred frequency of operation 102 at which the amplitude of the received signal 96 is at a maximum and which variations in the frequency 98 of operation of the signal transmission system will result in minimal changes in the received amplitude 96 due to the minimum of slope of the curve 100. However, as the coils of the transmitting antenna 14 and the receiving antenna 20 are spaced at closer than the critical coupling distance or if the resonant frequencies are not identical, the curve in Figure 5 differs. An example of the curve representing the received amplitude 96 as a function of the frequency 98 of operation is shown by curve 104. Instead of a single peak at frequency 102, the curve 104 demonstrates that there are really two amplitude peaks occurring on either side of frequency of 102. Note that the receiving amplitude 96 at the maximum in curve 104 is virtually identical to the received amplitude 96 of curve 100. That amplitude, however, is achieved at a different frequency of oscillation. Thus, the preferred frequency of oscillation for curve 104 differs from the frequency of operation for curve 100. With the transmitter 12 and receiver 18 of the present invention coupled with the transmitting antenna 14 and receiving antenna 20, the frequency of oscillation of the oscillator of transmitter 12 will automatically track to either frequency 106 or 108 maintaining the virtually identical received amplitude 96, albeit at a different frequency.

Thus, the unique transmitter 12, transmitting antenna 14 positioned in conjunction with receiving antenna 20 and receiver 18 provide a unique signal transmission system in which the received amplitude 96 of the signal at the receiver 18 is relatively independent of variations in coil misalignment between the transmitting antenna 14 and receiving antenna 20 as well as mismatches between the resonant frequencies of the transmitting antenna 14 and the receiving antenna 20. This is because the frequency of oscillation of the oscillator of transmitter 12 will automatically track to the proper frequency due to the resonant load which that oscillator drives.

Thus, it has been seen that there has been shown and described a novel signal transmission system. It is to be recognized and understood, however, that various changes, modifications and substitutions in the form and details of the present invention can be made by those skilled in the art without departing from the scope of the following claims.

## Claims

1. A signal transmission system (10) for use in transmitting a signal across a barrier (16), having a radio frequency receiver (18) for use on one side of said barrier (16); a receiving resonant antenna (20) for use on said one side of said barrier (16) being operatively coupled to said radio frequency receiver (18), said receiving resonant antenna (20) having a resonant frequency; a transmitting resonant antenna (14) for use on the opposite side of said barrier (16) nearby said receiving resonant antenna (20), said transmitting resonant antenna (14) having a resonant frequency; said resonant frequency of said receiving resonant antenna (20) being relatively close to said resonant frequency of said transmitting resonant antenna (14); and having a radio frequency transmitter (12) for use on said opposite side of said barrier (16) being operatively coupled to said transmitting resonant antenna (14), said radio frequency transmitter (12) having an oscillator (50-72) having a frequency of oscillation driving a resonant load where said resonant load includes said transmitting resonant antenna (14) inductively coupled with said receiving resonant antenna (20); characterized by:
   means for tracking the frequency of oscillation of said oscillator (50-72) to the resonant frequency of said resonant load and maintaining a virtually identical amplitude of the signal provided to the receiver by said transmission system; whereby signal transmission by said signal transmission system (10) is relatively independent of the distance between said transmitting resonant antenna (14) and of said receiving resonant antenna (20), of radial misalignment between said transmitting resonant antenna (14) and said receiving resonant antenna (20), and resonant frequency mismatch of said transmitting resonant antenna (14) and said receivingresonant antenna (20).

2. A signal transmission system (10) as claimed in Claim 1 for use in a transcutaneous environment, wherein:

6

said radio frequency receiver (18) is for subcutaneous use;
said receiving resonant antenna (20) is for subcutaneous use;
said transmitting resonant antenna (14) is for supercutaneous use; and
said radio frequency transmitter (12) is for supercutaneous use.

3. A signal transmission system (10) as claimed in Claims 1 or 2 wherein said resonant frequency of said receiving resonant antenna (20) is within ten percent of said resonant frequency of said transmitting resonant antenna (14).

4. A signal transmission system (10) as claimed in Claims 1 or 2 wherein said frequency of oscillation of said oscillator (50-72) is primarily determined by said resonant frequency of said resonant load.

5. A signal transmission system (10) as claimed in Claims 1 or 2 wherein said transmitting resonant antenna (14) comprises a parallel coupled inductor (22) and capacitor (24) combination.

6. A signal transmission system (10) as claimed in Claim 5 wherein said transmitter (12) has only one resonant circuit.

7. A signal transmission system (10) as claimed in Claim 5 wherein said radio frequency transmitter (12) has a pair of transistors (50-52) which are alternatively active at a rate which is a function of the resonant frequency of said transmitting resonant antenna (14) as influenced by said receiving resonant circuit.

**Revendications**

1. Système de transmission de signaux (10) utilisé pour transmettre un signal à travers une barrière (16), ayant un récepteur à fréquence radio (18) disposé sur un premier côté de la barrière (16), une antenne résonnante réceptrice (20), disposée sur ce premier côté de la barrière (16) et étant fonctionnellement couplée à ce récepteur à fréquence radio (18), l'antenne résonnante réceptrice (20) ayant une fréquence de résonnance, une antenne résonnante émettrice (14) disposée sur le côté opposé de la barrière (16) à proximité de l'antenne résonnante réceptrice (20), cette antenne résonnante émettrice (14) ayant une fréquence de résonnance, la fréquence de résonnance de l'antenne résonnante réceptrice (20) étant relativement proche de la fréquence de résonnance de l'antenne résonnante émettrice (14), et ayant un émetteur à fréquence radio (12), disposé sur ce côté opposé de la barrière (16) et étant en fonctionnement couplé à l'antenne résonnante émettrice (14), cet émetteur à fréquence radio (12) ayant un oscillateur (50-72) ayant une fréquence d'oscillations entraînant une charge résonnante constituée par l'antenne résonnante émettrice (14) inductivement couplée à l'antenne résonnante réceptrice (20), caractérisé par
   - des moyens pour aligner la fréquence de l'oscillation de cet oscillateur (50-72) sur la fréquence de résonnance de la charge résonnante et pour maintenir une amplitude pratiquement identique des signaux envoyés au récepteur par le système de transmission, d'où il résulte que la transmission des signaux par ce système de transmission de signaux (10) est relativement indépendante de la distance entre l'antenne résonnante émettrice (14) et l'antenne résonnante réceptrice (20), du défaut d'alignement radial entre l'antenne résonnante émettrice (14) et l'antenne résonnante réceptrice (20), et du désaccord des fréquences de résonnance de l'antenne résonnante émettrice (14) et de l'antenne résonnante réceptrice (20).

2. Système de transmission de signaux (10) selon la revendication 1, utilisé dans un environnement transcutané, dans lequel :
   - le récepteur à fréquence radio (18) est prévu pour une utilisation sous-cutanée ;
   - l'antenne résonnante réceptrice (20) est prévue pour une utilisation sous-cutanée ;
   - l'antenne résonnante émettrice (14) est prévue pour être utilisée sur la peau ; et
   - l'émetteur à fréquence radio (12) est prévu pour être utilisé sur la peau.

3. Système de transmission de signaux (10) selon la revendication 1 ou la revendication 2, dans lequel la fréquence de résonnance de l'antenne résonnante réceptrice (20) est égale, à plus ou moins 10% près, à la fréquence de résonnance de l'antenne résonnante émettrice (14).

4. Système de transmission de signaux (10) selon la revendication 1 ou la revendication 2, dans lequel la fréquence d'oscillation de l'oscillateur (50-72) est déterminée en premier lieu par la fréquence de résonnance de la charge résonnante.

5. Système de transmission de signaux (10) selon la revendication 1 ou la revendication 2, dans lequel l'antenne résonnante émettrice (14) est une combinaison d'une self (22) et d'un condensateur (24) couplés en parallèle.

6. Système de transmission de signaux (10) selon la revendication 5, dans lequel l'émetteur (12) a seulement un circuit résonnant.

7. Système de transmission de signaux (10) selon la revendication 5, dans lequel l'émetteur à fréquence radio (12) a deux transistors (50-52) qui sont alternativement actifs à une fréquence fonction de la fréquence de résonnance de l'antenne résonnante émettrice (14) telle qu'influencée par le circuit résonnant récepteur.


**Ansprüche**

1. Signalübertragungssystem (10) zum Übertragen eines Signals über eine Sperre (16), mit einem für die Verwendung auf der einen Seite der Sperre (16), bestimmten Hochfrequenzempfänger (18); einer für die Verwendung auf der genannten einen Seite der Sperre (16) bestimmten, resonanzfähigen Empfangsantenne (20), die mit dem Hochfrequenzempfänger (18) in Wirkungsbeziehung steht und eine Resonanzfrequenz hat; einer für die Verwendung auf der entgegengesetzten Seite der Sperre (16) in der Nähe der abgestimmten Resonanzantenne (20) bestimmten, resonanzfähigen Sendeantenne (14), die eine Resonanzfrequenz hat, wobei die Resonanzfrequenz der resonanzfähigen Empfangsantenne (20) relativ nahe bei der Resonanzfrequenz der resonanzfähigen Sendeantenne (14) liegt; und mit einem für die Verwendung auf der entgegengesetzten Seite der Sperre (16) bestimmten Hochfrequenzsender (12), der mit der resonanzfähigen Sendeantenne (14) in Wirkungsbeziehung steht und der einen Oszillator (50-72) mit einer zum Aussteuern einer resonanzfähigen Last bestimmten Schwingungsfrequenz besitzt, wobei die resonanzfähige Last die resonanzfähige Sendeantenne (14) aufweist, die mit der resonanzfähigen Empfangsantenne induktiv gekoppelt (20) ist, dadurch gekennzeichnet, daß durch: eine Einrichtung zum Nachführen der Schwingungsfrequenz des Oszillators (50-72) entsprechend der Resonanzfrequenz der resonanzfähigen Last und zum Aufrechterhalten einer oraktisch identischen Amplitude des von dem Übertragungssystem an den Empfänger abgegebenen Signals; wobei die Signalübertragung durch das Signalübertragungssystem (10) von dem Abstand zwischen der resonanzfähigen Sendeantenne (14) und der resonanzfähigen Empfangsantenne (20), von einem radialen Fluchtungsfehler zwischen der resonanzfähigen Sendeantenne (14) und der resonanzfähigen Empfangsantenne (20) und von einer Nichtübereinstimmung zwischen den Resonanzfrequenzen der resonanzfähigen Sendeantenne (14) und der resonanzfähigen Empfangsantenne (20) relativ unabhängig ist.

2. Signalübertragungssystem (10) nach Anspruch 1 für die Verwendung in einer transkutanen Umgebung, dadurch gekennzeichnet, daß
der Hochfrequenzempfänger (18) für eine subkutane Verwendung bestimmt ist;
die resonanzfähige Empfangsantenne (20) für eine subkutane Verwendung bestimmt ist;
die resonanzfähige Sendeantenne (14) für eine superkutane Verwendung bestimmt ist; und
der Hochfrequenzsender (12) für eine superkutane Verwendung bestimmt ist.

3. Signalübertragungssystem (10) nach Anspruch 1 oder 2, dadurch gekennzeichnet, daß die Resonanzfrequenz der resonanzfähigen Empfangsantenne (20) von der Resonanzfrequenz der resonanzfähigen Sendeantenne (14) um höchstens 10% abweicht.

4. Signalübertragungssystem (10) nach Anspruch 1 oder 2, dadurch gekennzeichnet, daß die Schwingungsfrequenz des Oszillators (50-72) in erster Linie durch die Resonanzfrequenz der resonanzfähigen Last bestimmt wird.

5. Signalübertragungssystem (10) nach Anspruch 1 oder 2, dadurch gekennzeichnet, daß die resonanzfähige Sendeantenne (14) einen Induktor (22) und einen Kondensator (24) besitzt, die parallelgeschaltet sind.

6. Signalübertragungssystem (10) nach Anspruch 5, dadurch gekennzeichnet, daß der Sender (12) nur einen Resonanzkreis besitzt.

7. Signalübertragungssystem (10) nach Anspruch 5, dadurch gekennzeichnet, daß der Hochfrequenzsender (12) zwei Transistoren (50-52) aufweist, die im Wechsel mit einer Frequenz aktiv sind, die eine Funktion der von dem resonanzfähigen Empfangskreis beeinflußten Resonanzfrequenz der resonanzfähigen Sendeantenne (14) ist.

**FIG.1**

**FIG.2**

FIG.3

FIG.4
PRIOR ART

RECEIVER
AMPLITUDE
96

ANTENNA SPACING -95

FIG.5

RECEIVER
AMPLITUDE
96

FREQUENCY -98